# EUROPEAN PATENT SPECIFICATION

(11) **EP 4 452 064 B1**
(45) Date of publication and mention of the grant of the patent: **12.08.2026**
(21) Application number: 22840716.9
(22) Date of filing: 20.12.2022
(51) Int. Cl.: A61B 5/145, A61B 5/01, A61B 5/00

(54) **BODY WEARABLE ANALYTE SENSOR SYSTEM WITH INFRARED TEMPERATURE SENSOR DEVICE**
AM KÖRPER TRAGBARES ANALYTSENSORSYSTEM MIT KONTAKTLOSEM TEMPERATURSENSOR
SYSTÈME DE CAPTEUR D'ANALYTE VESTIMENTAIRE AVEC CAPTEUR DE TEMPÉRATURE SANS CONTACT

(30) Priority: 22.12.2021 EP 21216887
(43) Date of publication of application: 30.10.2024
(73) Proprietor: F. Hoffmann-La Roche AG, 4070 Basel (CH); Roche Diabetes Care GmbH, 68305 Mannheim (DE)
(72) Inventor: THOES, Bruno, 68305 Mannheim (DE); BAUMANN, Edgar, 68305 Mannheim (DE); WETTENGEL, Klaus, 68305 Mannheim (DE)
(74) Representative: Freiherr von Campenhausen, Harald
(86) International application number: PCT/EP2022/086988
(87) International publication number: WO 2023/118139

(56) References cited:
- EP-B1- 2 345 366
- EP-B1- 2 557 987
- US-A1- 2013 197 847
- US-A1- 2020 275 894

## Description

The present invention provides an analyte sensor system which comprises a body wearable analyte sensor device, comprising a transcutaneous analyte sensor, a housing comprising a lower side which can be attached to the skin of a patient, an infrared (IR) temperature sensor device which is configured to detect the temperature (i) of the lower side of the housing, or (ii) of the skin through a hole comprised by the lower side of the housing, respectively, wherein the IR temperature sensor device faces without contact (i) the lower side of the housing, or (ii) the skin, respectively, and an electronic unit comprising a processor configured to receive analyte sensor signals from the transcutaneous analyte sensor and temperature sensor signals from the IR temperature sensor device. In addition, a method of determing an analyte concentration using an analyte sensor system of the invention is provided.

Monitoring certain body analytes plays an important role in the prevention and treatment of various diseases. Without restricting further possible applications, the invention will be described in the following text with reference to blood-glucose monitoring. However, the invention can also be applied to other types of analytes.

Blood glucose monitoring may be performed by using optical or electrochemical analyte sensors. Examples of electrochemical sensors for measuring glucose, specifically in blood or other body fluids, are known.

In addition to so-called spot measurements, in which a sample of a body fluid is isolated from a user in a targeted fashion and examined with respect to the analyte concentration, continuous determinations are increasingly becoming established. Thus, in the recent past, continuous measuring of glucose in the interstitial tissue (also referred to as continuous monitoring, CM) for example has been established as another important method for managing, monitoring and controlling a diabetes state.

In the process, the active sensor region of the analyte sensor system is applied directly to the detection site, which is generally arranged in the interstitial tissue, and, for example, converts glucose into electrical charge by using an enzyme (e.g. glucose oxidase, GOD), which charge is related to the glucose concentration and can be used as a detection variable.

Hence, hitherto known analyte sensor systems for continuous monitoring typically are transcutaneous systems or subcutaneous systems, wherein both expressions, in the following, will be used equivalently. This means that the actual sensor or at least a measuring portion of the sensor is arranged under the skin of the user. However, an evaluation and control part of the system is generally situated outside of the body of the user, outside of the human or animal body. In the process, the sensor is generally applied using an insertion instrument, which is likewise known in the art.

The sensor typically comprises a substrate, such as a flat substrate, onto which an electrically conductive pattern of electrodes, conductive traces and contact pads may be applied. In use, the conductive traces typically are isolated by using one or more electrically insulating materials. The electrically insulating material typically further also acts as a protection against humidity and other detrimental substances and, as an example, may comprise one or more cover layers such as resists.

As outlined above, in transcutaneous systems, a control part is typically required, which may be located outside the body tissue and which has to be in communication with the sensor. Typically, this communication is established by providing at least one electrical contact between the sensor and the control part, which may be a permanent electrical contact or a releasable electrical contact. Examples of electrical contacts for contacting a triangular assembly of contact pads are known in the art. Other techniques or providing electrical contacts, such as by appropriate spring contacts, are also generally known and may be applied.

In order to avoid detrimental effects of the aggressive environment onto the conductive properties of the electrical contact, the region of the electrical contact is typically encapsulated and protected against humidity. Generally, encapsulations of electrical locks and contacts by using appropriate seals is known.

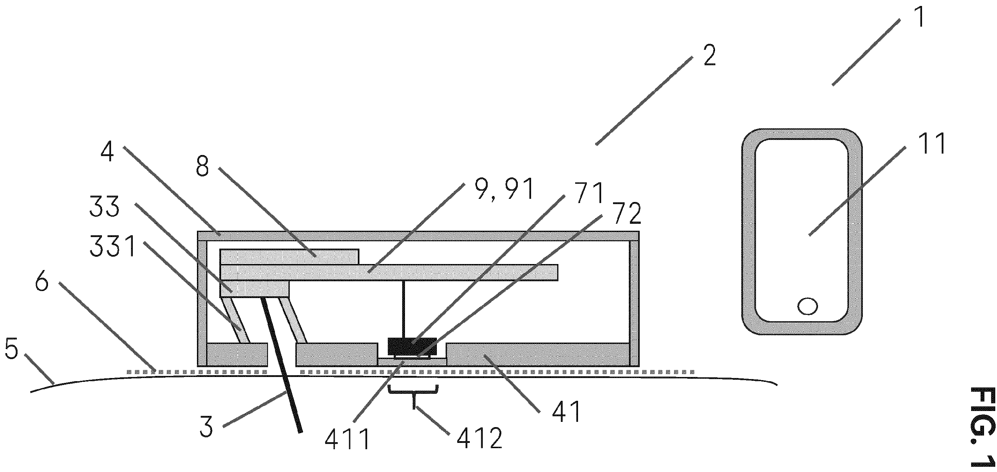

It is also generally known that the analyte detection of glucose analyte sensor and in particular the detection of the analyte concentration may be influenced by the temperature of the sensor. As a consequence, in order to achieve consistent determination of, e.g. the analyte concentration at varying temperatures of the body wearable analyte sensor device attached to the skin of the patient during use, the body wearable analyte sensor device is often equipped with a contact temperature sensor positioned in contact with a circuit board inside the housing of the body wearable analyte sensor device or in contact with the side of this housing attached to the skin of the patient (e.g. see Fig.1) and the contact temperature sensor signals are included as a factor in addition to the analyte sensor signals from the analyte sensor in the determination of the analyte value such as the analyte concentration.

US20210259586A1 discloses a non-invasive optical glucose sensor system comprising a housing attached to the skin, a total reflection member configured to totally reflect an incoming probe beam in a state being in contact with a detected object, a glucose sensor, a temperature sensor which is a sensor in contact with the object to be detected, a processor, and a temperature adjuster configured to maintain, to a predetermined temperature, a temperature of a contact region of the total reflection member with the detected object (e.g. skin) based on the temperature detection of the temperature sensor. In other words, the temperatures is detected in order to adjust the temperature of the contact region of the total reflection member but the temperature mearsurement is not used to determine a sensed analyte value.

Despite the advantages and the progress achieved by the above-mentioned developments, specifically in the field of continuous monitoring technology, some significant technical challenges remain. For example, the attachment of the contact temperature sensor to the housing of the body wearable analyte sensor device is associated with complex design and high production costs, e.g. because it is associated with additional wiring between the circuit board and the contact temperature sensor. Moreover, if the contact temperature sensor is attached to the circuit board heating up of the board may adversely affect the temperature detection by the contact temperature sensor that is intended to approximate the temperature of the skin essentially without influence from the temperature of the circuit board. The documents US 2020/275894 A1 and EP 2 557 987 B1 are relevant prior art for the present patent application.

### Problem to be solved

It is therefore an objective of the present invention to provide an analyte sensor system and a method of determing an analyte concentration using an analyte sensor system, which at least partially avoid the shortcomings described above.

### Summary of the invention

At least one of the above objects is solved by the provision of the analyte sensor systems specified in the claims and herein below. Further advantages, features and technical effects of the present invention are apparent from the following description of embodiments and the accompanying figures.

According to a first aspect it is provided an analyte sensor system which comprises a body wearable analyte sensor device, comprising a transcutaneous analyte sensor, a housing comprising a lower side which can be attached to the skin of a patient, an infrared (IR) temperature sensor device which is configured to detect the temperature (i) of the lower side of the housing, or (ii) of the skin through a hole comprised by the lower side of the housing, respectively, wherein the IR temperature sensor device faces without contact (i) the lower side of the housing, or (ii) the skin, respectively, and an electronic unit comprising a processor configured to receive analyte sensor signals from the transcutaneous analyte sensor and temperature sensor signals from the IR temperature sensor device. In addition, a method of determing an analyte concentration using an analyte sensor system of the invention is disclosed.

This invention is associated with the surprising effect that the impact of the skin temperature on the calculation of the analyte value can be assessed and corrected for more accurately compared to hitherto known systems because the detection of the IR temperature tempeature sensor device directly detects the temperature of the skin or of the side of the housing attached to the skin without being significantly impacted by the temperature of the IR temperature sensor device itself. This effect therefore also provides for more flexibility in the technical design of the analyte system because the IR temperature sensor device does not need to be positioned on the housing side attached to the skin but can also be positioned on an other part inside the housing, such as on a circuit board, where the IR temperature sensor device faces the side of the housing attached to the skin. This way also glueing the IR temperature sensor device to the housing side attached to the skin and the complex wiring of the IR temperature sensor device with the circuit board can be avoided that is used in contact temperature sensors based analyte sensor system (see e.g. Fig.1). Also in view of the above this invention is associated with the surprising effect that the production costs and time can be lowered compared to hitherto known systems.

Within the present invention the term "analyte" shall mean an arbitrary element, component or compound which may be present in the body fluid and the presence and/or the concentration of which may be of interest for the user, the patient or medical staff such as a medical doctor. Preferably, the analyte may be or may comprise an arbitrary chemical substance or chemical compound which may take part in the metabolism of the user or the patient, such as at least one metabolite. As an example, the at least one analyte may be selected from the group consisting of glucose, cholesterol, triglycerides, lactate. Additionally or alternatively, however, other types of analytes may be used and/or any combination of analytes may be determined. The detection of the at least one analyte specifically may be an analyte-specific detection. Preferably, the analyte is glucose.

Within the present invention the term "sensor" shall mean an arbitrary element which is configured to perform a process of the detection and/or which is configured to be used in the above-mentioned process of the detection. Thus, the sensor specifically may be configured to determine the concentration of the analyte and/or a presence of the analyte. The sensor may particularly be a "transcutaneous analyte sensor".

As used herein, the term **"transcutaneous analyte sensor"** shall mean a sensor which is configured to detect an analyte and to be fully or at least partly arranged within the interstitial fluid of skin or of under the skin of the patient. For this purpose, the analyte sensor generally may be dimensioned such that a transcutaneous insertion is feasible, such as by providing a width in a direction perpendicular to an insertion direction of no more than 5 mm, preferably of no more than 2 mm, more preferably of no more than 1.5 mm. The sensor may have a length of less than 50 mm, such as a length of 30 mm or less, e.g. a length of 5 mm to 30 mm. It shall be noted, however, that other dimensions are feasible. In order to further render the sensor to be usable as a transcutaneous sensor, the sensor may fully or partially provide a biocompatible surface, i.e. a surface which, at least during durations of use, do not have any detrimental effects on the user, the patient or the body tissue. As an example, the transcutaneous sensor may fully or partially be covered with at least one biocompatible membrane, such as at least one polymer membrane or gel membrane which is permeable for the at least one analyte and/or the at least one body fluid and which, on the other hand, retains sensor substances such as one or more test chemicals within the sensor and prevents a migration of these substances into the body tissue. The sensor preferably may be an electrochemical analyte sensor. As used herein, an electrochemical analyte sensor generally is a sensor which is configured to conduct an electrochemical detection in order to detect the at least one analyte contained in the body fluid, preferably in the insterstitial fluid of the skin. The term "electrochemical detection" refers to a detection of an electrochemically detectable property of the analyte, such as an electrochemical detection reaction. Thus, for example, the electrochemical detection reaction may be detected by comparing one or more electrode potentials, as further discussed below. The electrochemical sensor specifically may be configured to and/or may be usable to generate at least one electrical sensor signal which directly or indirectly indicates the presence and/or the extent of the electrochemical detection reaction, such as at least one current and/or at least one voltage. For this purpose, as will be outlined in further detail below, the at least one electrochemical analyte sensor provides two or more electrodes, which also are referred to as a sensor electrodes. The detection may be analyte-specific. The detection may be a qualitative and/or a quantitative detection.

Within the present invention the term **"analyte sensor system"** shall mean a kit of parts that comprises at least a body wearable analyte sensor device and an an electronic unit. Additional components which may be part of the analyte sensor system include at least one of (1) an insertion device for inserting the analyte sensor into the skin, (2) a cradle configured to mechanically attach to the skin with its proximal side via a plaster bonded to the cradle and to the body wearable analyte sensor with its distal site, (3) at least one accessory such as a plaster, a cover, a package, a battery charger, an electronic consumer device adepted to communicate wirelessly or in a wired fashion with and/or in electrical connection with the body wearable analyte sensor device and/or with the electronic unit, the electronic consumer device may be a smartphone, a pager, a smartwatch or any other electronic wearable device.

Within the present invention the term **"body wearable analyte sensor device"** shall mean that the analyte sensor device can be mounted on the body of the patient, preferably on the skin on any body part of the patient such as on the belly, an arm, a leg, the hip, the abdomen, the neck, an ear, the face, etc.

Within the present invention the term **"analyte sensor device"** shall mean a group of at least two elements which may interact in order to fulfill at least one common function. The at least two components may be handled independently or may be coupled, connectable or integratable in order to form a common component. Thus, an **"analyte sensor device"** generally refers to a group of at least two elements or components which are capable of interacting in order to perform at least one analyte sensor function, in the present case in order to perform at least one detection of an analyte in the body fluid and/or in order to contribute to the at least one detection of an analyte in the body fluid. The analyte sensor device may particularly be a device wherein the sensor is wholly or at least partly arranged within the body tissue of the patient. At least one component of the sensor system may be wholly or partly outside of the body tissue, for example the housing. During use the distal sensing part of the analyte sensor protrudes from the housing and is positioned in the skin or under the skin of the patient whereas the proximal non-sensing part of the analyte sensor connects the sensing part with the interior of the housing, specifically the distal part is electrically connected to the circuit board of the body wearable analye sensor device.

Within the present invention the term **"housing"** shall mean an arbitrary element which is configured to surround one or more elements in order to provide one or more of a mechanical connection protection, an environmental protection against moisture and/or ambient atmosphere, a shielding against electromagnetic influences or the like. Specifically, the housing may be configured to shield one or more elements of the body wearable analyte sensor device from external influences like moisture and/or mechanical stress. The housing may be a watertight housing having an essentially round shape. Further, the housing may have an essentially flat lower side facing the skin. The housing, in general, may comprise one or more parts. The housing can be made of any material conventionally used to manufacture body worn medical devices such as from plastic or metal, preferably from plastic. Examples of suitable plastics are polycarbonate and polyethylen HD.

Within the present invention the term **"lower side of the housing"** shall mean the side of the housing attached to the skin either directly with a plaster or indirectly via a cradle where the housing can be mounted on. Preferably the side is directly attached to the skin with a plaster. The lower side is intended to mean the wall of the housing amongst the housing walls that is attached to the skin and/or which is positioned closest to the skin.

Within the present invention the term **"patient"** shall mean a human being or an animal, independent from the fact that the human being or animal, respectively, may be in a healthy condition or may suffer from one or more diseases. As an example, the patient or the user may be a human being or an animal suffering from diabetes. However, additionally or alternatively, the invention may be applied to other types of users or patients or diseases.

Within the present invention the term **"plaster"** shall mean an attachment component which is capable of connecting the body wearable analyte sensor device to the skin. The plaster comprises at least one adhesive surface and/or at least one adhesive strip that can be attached to the skin. The opposite side of the plaster can be attached to the lower side of the housing or to a cradle with an adhesive surface, though this attachment can also be a welded / covalent connection.

In a preferred embodiment of the analyte sensor system of the invention the housing comprises a contact temperature sensor, wherein the contact temperature sensor (i) is part of the IR temperature sensor device, or (ii) is not part of the IR temperature sensor device and in contact with a part of the housing carrying the IR temperature sensor device or with the circuit board. Optionally, the contact temperature sensor is selected from the group consisting of a thermoelement, a thermistor, and a resistance temperature dectector. Such contact sensors are generally known in the art. Preferably, when the contact temperature sensor is part of the IR temperature sensor device it is in contact with the IR temperature sensor device and will therefore detect the temperature of the IR temperature sensor device which will essentially be the same temperature as the temperature of the IR temperature sensor.

Within the present invention the term **"detect the temperature of the lower side of the housing"** shall mean that the IR temperature sensor device detects, ascertains and/or quantifies the temperature of the lower side of the housing. In particular, the IR temperature sensor device detects the temperature of the internal surface of the lower side of the housing facing the sensor, preferably in the temperature detection area of the lower side of housing. It is clear to the skilled person that such detected temperature will provide a reliable approximation of the temperature of the skin given that the skin temperature will cause the attached lower side of the housing to adapt to the skin temperature. The accuracy of that approximation increases as the thickness of the lower side or at least the thickness in the detection area of the lower side is reduced relative to the thickness of other areas of the lower side and/or the heat conductance properties of the material used for the lower side of the housing increases. Preferably, the IR temperature sensor device faces without contact the lower side of the housing, and the lower side of the housing in a temperature detection area detected by the IR temperature sensor device has a reduced thickness relative to the thickness of other areas of the lower side .

Within the present invention the term **"detect the temperature of the skin"** shall mean that the IR temperature sensor device detects, ascertains and/or quantifies the temperature of skin which is preferably not covered by any lower side and preferably also not by any other part such as a plaster or cradle so that the IR temperature sensor device can direcly detect the temperature of the unobstructed skin, preferably in the detection area which is located under a hole in the lower side of the housing which exposes the skin to direct and unobstructed detection by the IR temperature sensor device. Preferably, the IR temperature sensor device is configured to detect the temperature of the skin through a hole comprised by the lower side of the housing. Preferably, the IR temperature sensor device faces without contact the skin, wherein the IR temperature sensor device does not contact the lower side of the housing and also not the skin, wherein the lower side of the housing comprises the hole defining a temperature detection area of the skin configured such that the temperature of the skin below the hole can be detected by the IR temperature sensor device.

Within the present invention the term **"IR temperature sensor device faces without contact the lower side of the housing"** shall mean that essentially no physical object is located between the IR temperature sensor device and the lower side of the housing. Preferably, the term means that no physical object is located between the temperature representing sensor stimulus (e.g. IR radiation emitted from the lower side of the housing) detecting part of the IR temperature sensor device and the lower side of the housing, preferably the measuring area of the lower side of the housing, preferably the measuring area of the inner surface of the lower side of the housing. Preferably, the IR temperature sensor device does not contact the lower side of the housing and it also does not contact the skin.

In a preferred embodiment the housing of the body wearable analyte sensor device comprises an electronic unit which comprises a processor configured to receive analyte sensor signals from the analyte sensor and temperature sensor signals from the IR temperature sensor device. Preferably, the processor comprises a memory with instructions that can be executed by the processor to carry out process steps recited throughout the specification and the claims. The electronic unit is configured to controlling the detection of the analyte and/or for transmitting sensor analyte data to another component such as a display device configured to communicate with the body wearable analyte sensor device and/or to another electronic device that lacks a display. The electronic unit comprises at least two electrical contacts which are electrically connected to the contact pads of the sensor. The electronic unit also comprises the circuit board, preferably the printed circuit board, of the invention. Preferably, the IR temperature sensor device is mounted on a circuit board, preferably on a printed circuit board.

Within the present invention the term **"electronic unit"** shall mean a device having at least one electronic component. Specifically, the electronic unit may comprise at least one electronic component for one or more of processing of sensor signals, sensor, performing a voltage detection, performing a current detection, recording sensor signals, storing detection signals or detection data, transmitting sensor signals or detection data to another device. Other embodiments of the electronic components are feasible. The electronic unit specifically may comprise at least one circuit board having disposed thereon at least one electronics component, such as at least one active and/or at least one passive component. The electronic unit may further comprise at least one housing which fully or partially surrounds the electronics component. The electronic unit may further comprise at least one of an integrated circuit, a microcontroller, a computer or an application-specific integrated circuit (ASIC). The electronic unit may specifically be embodied as a transmitter or may comprise a transmitter, for transmitting data. Preferably, the electronic unit may be reversibly connectable to the body mount.

According to another embodiment the electronic unit is part of a transmitter unit component which is a component separte from the wearable analyte sensor device but which transmitter unit can be mechanically and electrically coupled to the wearable analyte sensor device. In this embodiment the circuit board is not comprised by the electronic unit but by the body wearable analyte sensor device. The electronic unit is configured to receive analyte sensor data from the processor of the circuit board both comprised by the body wearable analyte sensor device. The electronic unit is also configured to communicate the received analyte sensor data to a display device.

In another embodiment of the analyte sensor system of the invention the IR temperature sensor device is mounted on a circuit board, preferably on a printed circuit board.

Preferably, the analyte sensor system of the invention further comprises a display device configured to communicate with the body wearable analyte sensor device.

Within the present invention the term **"IR temperature sensor device**" shall mean a temperature sensor device that is configured to detect the temperature of an object which is not in physical contact with the IR temperature sensor device, by detecting the infrared radition (IR)emitted by that object. To this end the IR temperature sensor device comprises an IR temperature sensor. The IR temperature sensor can be a passive infrared (PIR) sensor. Preferably, the IR temperature sensor device of the invention further comprises a contact temperature sensor which is part of the IR temperature sensor device. This contact temperature sensor is configured to detect the temperature of the IR temperature sensor device.

Alternatively, the contact temperature sensor is not part of the IR temperature sensor device and the contact temperature sensor is in contact with a part of the housing carrying the IR temperature sensor device or with the circuit board. In that case the contact temperature sensor is configured to detect the temperature of part of the analyte sensor system it is in contact with, i.e. a part of the housing carrying the IR temperature sensor device or the circuit board. As the skilled artisan will readily appreciate, in such scenario the contact temperature sensor will provide an accurate approximation of the temperature of the IR temperature sensor. This is particularly beneficial to calibrate the IR temperature sensor detection to compensate for changing temperatures of the IR temperature sensor device.

Preferably, the contact temperature sensor and the IR temperature sensor device, including its IR temperature sensor, are connected to a processor which is connected to a memory configured to store temperature sensor calibration data such as a temperature correction function. The processor is configured to determine the object temperature based on the received signals from the IR temperature sensor, the received signals from the contact temperature sensor and temperature sensor calibration data. Such calibration methods are readily known to the skilled person and exemplified in an Example below. Preferably, the processor determines the object temperature detected by the IR temperature sensor and outputs calibrated object temperature representing signals which are calibrated to compensate for changing temperatures of the IR temperature sensor device, and preferably its IR temperature sensor.

The contact temperature sensor is part of the IR temperature sensor device; or the contact temperature sensor is not part of the IR temperature sensor device and in contact with a part of the housing carrying the IR temperature sensor device or with the circuit board; and wherein the processor is configured to calibrate the temperature sensor signals from the IR temperature sensor with the temperature sensor signals from the contact temperature sensor and temperature sensor calibration data to determine the temperature of the skin. Preferably the calibrated signals compensate for changing temperatures detected by the contact temperature sensor.

Preferably, IR temperature sensor device is an integral device comprising an IR temperature sensor, a contact temperature sensor, a processor with an associated memory storing temperature calibration data wherein the device is configured to determine the object temperature detected by the IR temperature sensor and to output object temperature representing signals which are calibrated to compensate for changing temperatures of the IR temperature sensor device, and preferably its IR temperature sensor.

Preferably, the IR temperature sensor device is mounted on or attached to the electronic unit or the circuit board. Preferably, the **IR temperature sensor** of the IR temperature sensor device is configured to detect the temperature of the lower side of the housing or of the skin, which the IR temperature sensor faces without contact (i) the lower side of the housing (41), or (ii) the skin, respectively. Preferably the temperature representing stimulus detected by the IR temperature sensor is infrared radiation emitted from the lower side of the housing and/or from the skin.

Such IR temperature sensor devices which comprise an IR temperature sensor and a contact temperature sensor and which generate signals from both temperature sensors that can be readily calibrated to represent the detected temperature of object are generally known. The calibration preferably compensates for changing temperatures of the IR temperature sensor device detected by the contact temperature sensor. Examples of such IR temperature sensor devices include for example the IR temperature sensor devices available from Melexis Technologies NV, Belgium such as the MLX90632 which comprises an IR temperature sensor, a contact temperature sensor and which is adapted to output signals from the IR temperature sensor and signals from the contact temperature sensor.

The housing further comprises a contact temperature sensor.

Within the present invention the term **"contact temperature sensor"** shall mean a sensor that detects the temperature of the sensor itsfelf and its environment such as the object where it is mounted on. Examples of contact temperature sensors are generally known to the skilled person and may be selected from the group consisting of a thermoelement, a thermistor, and a resistance temperature detector. Suc contact sensors are generally known.

In another embodiment of the analyte sensor system of the invention the contact temperature sensor contacts a part of the housing carrying the IR temperature sensor or the circuit board.

Within the present invention the term **"contact a part of the housing"** shall mean any form of mechanical, chemical connection between the contact temperature sensor and the part it is mounted on or attached to including a housing side, a circuit board, etc. The closer the distance is between the contact temperature sensor and the IR temperature sensor, the more accurate the contact temperature sensor can detect the temperature of the part the IR temperature sensor is attached to or the temperature of the IR temperature sensor itself. In a preferred embodiment this contact temperature sensor based temperature detection is taken into account or checked when determining the analyte concentration based on the analyte sensor signals by the transcutaneous analyte sensor and the temperature sensor signals by the IR temperature sensor. This way any adverse effect on the accuracy of the IR temperature sensor's temperature detection caused by changing temperatures or by an inappropriately high or low temperature of the IR temperature sensor itself or of the part or component it is attached to can be uncovered and corrected for in the determining of the analyte concentration.

In another embodiment of the analyte sensor system of the invention the IR temperature sensor device faces without contact the skin, wherein the IR temperature sensor device does not contact the lower side of the housing and also not the skin, wherein the lower side of the housing comprises the hole defining a temperature detection area of the skin configured such that the temperature of the skin below the hole can be detected by the IR temperature sensor device. The direct detection of the skin temperature that is not obscured by a housing wall is associated with the surprising advantage that the skin temperature can be determined more accurately and temporal inertia effect of the housing wall on the temperature detection is essentially absent, thus increasing the accuracy of dynamic temperature detection and determination over time. This in turn, improves the accuracy of analyte value determination.

Within the present invention the term **"hole defining a temperature detection area of the skin"** shall mean an opening in the lower side of the housing that exposes the skin underneath the hole to the unobstructed access by the IR temperature sensor. The size and shape of the hole can have any kind of shape that the skilled person can readily determine bearing in mind measurement requirements determined by the detection field, area or angle of the IR temperature tempeature sensor device , requirements for avoiding ingress of dirt, fluid or humidity, etc.. Preferably, the hole has a circular, elipsoid, square or rectangular shape and preferably a maximum diameter of 0.5 to 3 cm, 0.5 to 2 cm, 0.5 to 1 cm, or 1 to 2 cm.

In another embodiment of the analyte sensor system of the invention a detection cell comprises the IR temperature sensor, the lower side of the housing comprising the hole, and separating means, preferably separating walls, sealing off the detection cell from the remaining interior space of the housing.

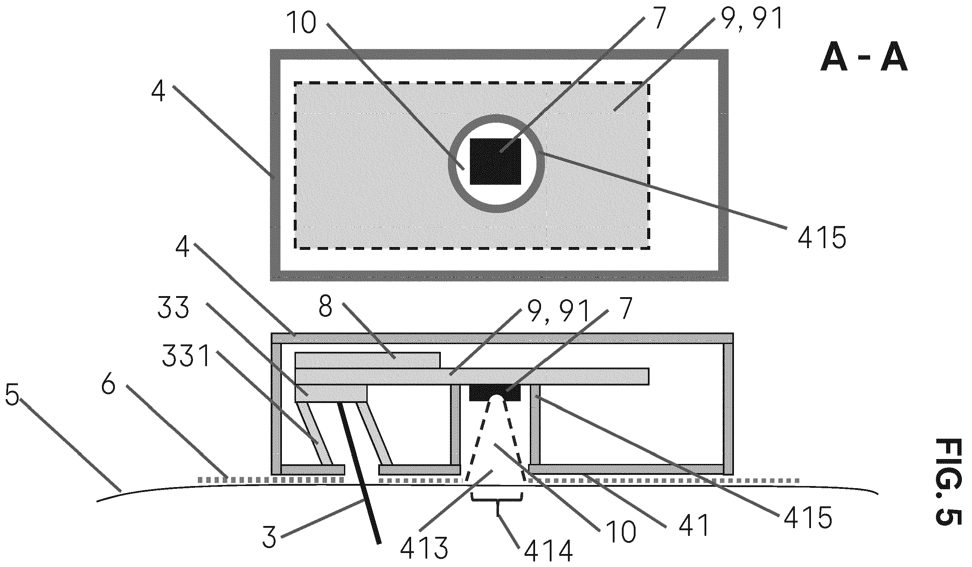

Within the present invention the term **"detection cell"** shall mean the space between the IR temperature sensor, the lower side of the housing comprising the hole, and separating means. This is for example illustrated in Figure 5. Preferably, the detection cell provides a sealing off of the detection cell vis-à-vis remaining interior space of the housing against ingress from fluid, humidity, and particles.

Within the present invention the term **"separating means"** shall mean a wall, a seal or a membrane that protects aggainst ingress from fluid, humidity, and particles.

In another embodiment of the analyte sensor system of the invention the lower side of the housing in a temperature detection area detected by the IR temperature sensor has a reduced thickness relative to the thickness of other areas of the lower side. The temperature detection area of the lower side can be the same part as the rest of the lower side or it can be a separate part which is integrated or connected to the rest of the lower side. If it is a separate part, temperature detection area can be made of the same or of different material than the rest of the lower side.

The thickness of the lower side of the housing ranges from 0.03-0.5 cm, preferably from 0.03-0.3 cm, preferably from 0.03-0.1 cm. Within the present invention the term **"reduced thickness relative to the thickness of other areas of the lower side"** shall mean that the reduced thickness ranges from ranges from 0.1-0.7 cm, preferably from 0.1-0.5 cm, preferably from 0.1-0.2 cm; preferably the thickness is reduced by 10 to 90%, preferable by 10 to 70%, preferable by 10 to 50%, preferable by 10 to 30%, relative to the thickness of other areas of the lower side.

The body wearable analyte sensor devices of the invention ranges in their maximum length of the housing from 0.5 to 5 cm, preferably from 0.5 to 3 cm, preferably from 0.5 to 2 cm. The maximum width of the housing ranges from 0.5 to 5 cm, preferably from 0.5 to 3 cm, preferably from 0.5 to 2 cm. The maximum length of the lower side of the housing ranges from 0.5 to 5 cm, preferably from 0.5 to 3 cm, preferably from 0.5 to 2 cm. The maximum width of the lower side of the housing ranges from 0.5 to 5 cm, preferably from 0.5 to 3 cm, preferably from 0.5 to 2 cm.

In another embodiment of the analyte sensor system of the invention the analyte system further comprises a display device configured to communicate with the body wearable analyte sensor device.

Within the present invention the term **"display device"** shall mean any electronic device that comprises as display, preferably a touch display, such as a smartphone, a pager, and a wearable including a smartwatch. The display device is configured to communicate wiressly or in a wired fashion with the body wearable analyte sensor device or a part thereof, with the electronic unit and/or with the transmitter unit.

In another embodiment of the analyte sensor system of the invention the analyte sensor is a glucose sensor. Within the present invention the term **"glucose sensor"** shall mean an optical or electrochemical analyte sensor configured to detect glucose. Such electrochemical biosensors for measuring glucose, specifically in blood or other body fluids, are generally known. The sensor may comprise an enzyme such as glucose oxidase and/ or glucose dehydrogenase.

Within the present invention the term **"receiving a temperature sensor signals"** shall mean that signals, preferably electrical signals, from a temperature sensor representing at least one parameter of the detected temperature, such as a qualitative or quantitive temperature parameter, which signals are received by an electrical or electronic component, such as by a wire or a processor, preferably by a processor or a memory.

In a preferred embodiment of the analyte sensor system of the invention the processor comprises a memory, wherein the processor is configured to receiving the temperature sensor signals from the IR temperature sensor device, and determining the temperature of the lower side of the housing, preferably of the internal surface of the lower side of the housing, or of the temperature of the skin based on the received temperature sensor signals from the IR temperature sensor and from the contact temperature sensor.

Within the present invention the term **"determining the temperature of the lower side of the housing"** shall mean a process of generating at least one representative result or a plurality of representative results indicating the temperature of the lower side of the housing. The temperature is preferably determined by the processor or by the electrical unit or a part thereof or by the circuit board or a part thereof. The temperature determination is based on received IR temperature sensor signals from a IR temperature sensor and optionally also based on contact temperature sensor signals from a contact temperature sensor.

Within the present invention the term **"internal surface of the lower side"** shall mean the surface of the lower side of the housing facing the IR temperature sensor. This is also the surface of the lower side that is oriented towards the interior of the housing, i.e. the surface of the lower side that is opposite of the other surface of the lower side which faces the plaster and the skin of the patient during use.

Within the present invention the term **"signals received from the contact temperature sensor"** shall mean asignals, preferably electrical signals, from a contact temperature sensor representing at least one parameter of the detected temperature, such as a qualitative of quantitive temperature parameter, which signals are received by an electrical or electronic component, such as by a wire or a processor, preferably by a processor or a memory.

In another embodiment of the analyte sensor system of the invention the the processor is further configured to receiving the analyte sensor signals from the transcutaneous analyte sensor, determining an analyte concentration based on the analyte sensor signals and the determined temperature, and communicating the analyte concentration to the display device.

Within the present invention the term **"determining an analyte concentration"** shall mean a process of generating at least one representative result or a plurality of representative results indicating the concentration of the analyte in the body fluid. The analyte concentration is preferably determined by the processor or by the electrical unit or a part thereof or by the circuit board or a part thereof. The analyte concentration determination is based on at least one received analyte sensor signals from the transcutaneous analyte sensor and may preferably be further based on other parameters such as analyte sensor calibration data, and the determined temperature.

Within the present invention the term **"communicating the analyte concentration to the display device"** shall mean the process of wired or wireless transmittion of a analyte concentration from the body wearable analyte sensor device, preferably from the processor to the display device, preferably via a transceiver comprised by the electrical unit and/or by the body wearable analyte sensor. The display device may then further process, display the analyte concentration on its display, and/or transmit the analyte concentration to another device via a network connection.

In another embodiment of the analyte sensor system of the invention the processor is further configured to comparing the determined temperature with a first and optionally also with a second predetermined reference temperature, and issueing a notification if (i) the determined temperature is above the first predetermined reference temperature, and (ii) optionally if the determined temperature is below the second predetermined reference temperature, wherein the first predetermined reference temperature is higher than the second predetermined reference temperature. Preferably the determined analyte concentration was detected within a predeterined time interval after the determined temperature was detected to be above the first predetermined reference temperature or optinally below the second predetermined reference temperature. Preferably, the predetermined time interval is up to 30 minutes, preferably up to 20 minutes, preferably up to 10 minutes, preferably up to 5 minutes, preferably up to 2 minutes, preferably up to 1 minute, preferably up to 30 seconds.

Preferably, the first predetermined reference temperature is between 40 to 50 °C , preferably between 40 to 45 °C , preferably between 40 to 42 °C . Preferably, the second predetermined reference temperature is between 0 to 15 °C , preferably between 5 to 15 °C , preferably between 10 to 15 °C , preferably between 5 to 10 °C . Preferably, the first predetermined reference temperature is between 40 to 45 °C , and the second predetermined reference temperature is between 5 to 10 °C.

Within the present invention the term **"predetermined reference temperature"** shall mean a reference temperature that is set and stored in the body wearable analyte sensor device or a part thereof, preferably in a memory. The predetermined reference temperature can be set and stored before hand over of the wearable analyte sensor device to the patient, such as during manufacturing or by a health care professional or by a patient before the wearable analyte sensor device is used on the body for the first time. Moreover, the predetermined reference temperature may also be configured during use of the body wearable analyte sensor device.

This embodiment is associated with the surprising advantage that it improves the safety of analyte measurement: the patient is informed if the determined temperature of the lower side of the housing, or the skin, respectively, is such that there is a risk that determination of the analyte concentration with the transcutanous analyte sensor is inaccurate and/or unreliable because the determined temperature is too high (i.e. above the first predetermined reference temperature) or too low (i.e. below the second predetermined reference temperature). A person skilled in the art may readily determine such temperature ranges for the lower side of the housing and the skin, respectively, where accurate and/or reliable operation of the transcutaneous analyte sensor is at risk such as based on regulatory requirements defined by a regulatory body such as the TÜV or Food and Drug Administration (FDA), or based on simulation or empirical testing of analyte concentration detection using an analyte sensor system of the invention in relation to determined temperatures in the skin (or a skin model system) exhibiting defined concentrations of the analyte of intered to be determined and also determined temperatures measured by the IR and contact temperature sensors of the invention. This in turn, will allow the skilled person to define adequate predetermined first and second reference temperatures.

Within the present invention the term **"issueing a notification"** shall mean that it is issued a message, alarm or signal that can be perceived by the patient. The notification preferably informs that the analyte concentration is at least one of the following: not reliable, cannot be trusted, cannot be used for treatment decisions such as decising on dosing or administration of a medicament, will not be stored, and will not be displayed.

In a preferred embodiment of the analyte sensor system of the invention the processor comprises further instructions which when executed cause the processor to comparing the determined temperature with a first and second predetermined reference temperature, and interrupting analyte detection by the transcutaneous analyte sensor, deleting or not storing data of the determined analyte concentration as long as the determined temperature is above the first predetermined reference temperature or below the second predetermined reference temperature, wherein the first predetermined reference temperature is higher than the second predetermined reference temperature. Preferably the determined analyte concentration was detected at a time when the determined temperature was detected to be above the first predetermined reference temperature or below the second predetermined reference temperature. Preferably, the interrupting analyte detection by the transcutaneous analyte sensor is interupted until the determined temperature is below the first predetermined temperature and above the second predetermined temperature.

In a preferred embodiment the determined temperature used for the termination of the analyte concentration is based on signals received by the processor by the IR temperature sensor comprised by the IR temperature sensor device, while the determined temperature received by the processor for interrupting analyte detection by the transcutaneous analyte sensor, deleting or not storing data of the determined analyte concentration is detected by a contact temperature sensor of the invention. In this embodiment the contact temperature sensor functions as a safety device to reveal if the temperature of or around the IR temperature sensor is still within or outside the temperature range where it operates properly in order to ensure that adequate temperature values can be provided by the IR temperature sensor for determinining the analyte concentration. Thus, in this embodiment the IR temperature sensor signals are not included in the determination of the analyte concentration if the temperature of or around the IR temperature sensor is outside the temperature range where it operates properly. In a preferred embodiment, in such cases the analyte sensor system will also not output an analyte concentration.

There is provided a method of determing an analyte concentration using an analyte sensor system of the invention as defined throughout the specification the claims and the figures, the method comprising the steps of: receiving the temperature sensor signals from the IR temperature sensor comprised by the IR temperature sensor device and from the contact temperature sensor, determining the temperature of the lower side of the housing, or of the temperature of the skin, based on the received temperature sensor signals from the IR temperature sensor and from the contact temperature sensor, receiving the analyte sensor signals from the transcutaneous analyte sensor within a predetermined time interval after receiving the temperature sensor signals from the IR temperature sensor and from the contact temperature sensor, determining an analyte concentration based on the analyte sensor signals and the determined temperature of the lower side of the housing, or of the temperature of the skin, and communicating the analyte concentration to the display device.

A method of determing an analyte concentration using an analyte sensor system of the invention is provided, the method further comparing the determined temperature with a predetermined reference temperature, and communicating to the display device a notification if the determined temperature is above of below a predetermined reference temperature.

### Figure Legend

The invention is further illustrated by the embodiments described and shown in the Figures and herein below.
- Figure 1: shows a schematic frontal view of an analyte sensor system with contact temperature sensor known in the art.
- Figure 2: shows a schematic frontal view of an analyte sensor system according to an embodiment of the present invention.
- Figure 3: shows a schematic frontal view of an analyte sensor system according to another embodiment of the present invention.
- Figure 4: shows a schematic frontal view of an analyte sensor system according to another embodiment of the present invention.
- Figure 5: shows a schematic bottom-up view of an analyte sensor system according to the embodiment shown in Figure 4.
- Figure 6: shows a temperature correction function described further in the Example, below.

### Detailed description of the invention

The following Figures 1 to 5 are only schematic figures that do not limit the proportion or dimension of the depicted embodiments or elements thereof.

**Figure 1** shows a schematic frontal view of an analyte sensor system with contact temperature sensor known in the art which comprises an analyte sensor system (1) for continuous glucose determination which comprises a body wearable analyte sensor device (2) that communicates with a display device (11). The body wearable analyte sensor device (2) can be mounted on the skin and maintained in the mounted position by way of a plaster (6). The body wearable analyte sensor device (2) comprises a transcutaneous analyte sensor (3) which when inserted into the skin can detect glucose in the interstitial fluid of or under the skin (5). The transcutaneous analyte sensor (3) can be held by transcutaneous analyte sensor holder (33) which in addition to providing mechanical support for the sensor also electrically couples the transcutaneous analyte sensor's (3) electrode wires with the circuit board (91) which can be a printed circuit board that also comprises a processor (8) with a memory and a transceiver (not shown) for communicating with the display device (11). To prevent that humidity, liquid or dirt enters the interior of the housing separating walls (331), that are preferably waterproof, separate the space around the transcutaneous analyte sensor (3) between the transcutaneous analyte sensor holder, the separating walls (331) and the lower side of the housing around the hole where the sensor protrudes from the housing in transcutaneous placement position from the remainder of the internal housing. The circuit board (91) is comprised by the electronic unit (9, not shown) and both receive their power from a connected battery (not shown) that can be located within the housing (4). The circuit board (91) is in wired connection with a contact temperature sensor (71) which is attached by glue (72) to the interior surface of the lower side of housing (41) which side faces away from the skin (5) of the patient. The housing (4) and specifically the outer surface of its lower side (41), i.e. opposite of the inner surface, is attached to the skin (5) via a plaster (6). To reduce the temperature inertia effect of the lower side of the housing on the temperature detection by the contact temperature sensor (71) the thickness of the lower side (41, 411), preferably of the wall of the lower side, is reduced in the temperature detection area of the lower side of housing (412). During use, the analyte concentration is determined based on the analyte sensor signals received by the processor (8) from the transcutaneous analyte sensor (3) and may also be based on the temperature signals received by the processor (8) from the contact temperature sensor (71).

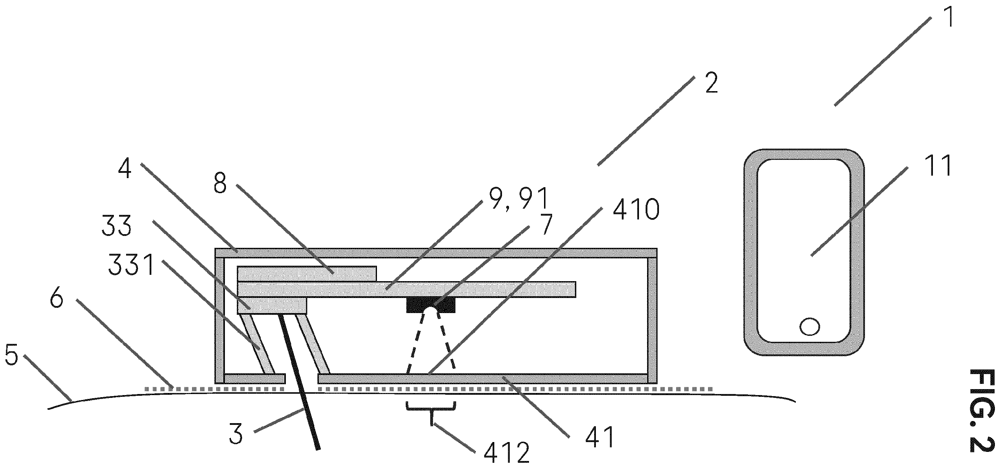

**Figure 2** shows a schematic frontal view of an analyte sensor system (1) according to an embodiment of the invention which comprises a body wearable analyte sensor device (2) and a display device (11) in communication with the body wearable analyte sensor device (2). The body wearable analyte sensor device (2), in turn, comprises a transcutaneous analyte sensor (3) which when inserted into the skin or under the skin can detect glucose in the interstitial fluid of the skin or under the skin. The transcutaneous analyte sensor (3) is held by transcutaneous analyte sensor holder (33) which in addition to providing mechanical support for the sensor also electrically couples the transcutaneous analyte sensor's (3) electrode wires with the circuit board (91, not shown) comprised by the electronic unit (9) which circuit board (91) can be a printed circuit board that also comprises a processor (8) with a memory (81, not shown) and a transceiver (not shown) for communicating with the display device (11). To prevent that humidity, liquid or dirt enters the interior of the housing separating walls (331), that are preferably waterproof, separate the space around the transcutaneous analyte sensor (3) between the transcutaneous analyte sensor holder, the separating walls (331) and the lower side of the housing around the hole where the sensor protrudes from the housing in transcutaneous placement position from the remainder of the internal housing. The circuit board (91) receives its power from a connected battery (not shown) that is also located within the housing (4). The circuit board (91) is in electrical connection with a IR temperature sensor device (7) attached to the circuit board (91). The housing (4) and specifically the outer surface of its lower side (41) is attached to the skin via a plaster (6). The IR temperature sensor device (7) is configured to detect the temperature of the internal surface of the lower side of the housing (41). The IR temperature sensor device (7) faces without contact the lower side of the housing (41), preferably the internal surface of the lower side of housing opposite of the surface facing the skin (410) and detects the temperature in the temperature detection area of the lower side of housing (412). To reduce the temperature inertia effect of the lower side of the housing (41) on the temperature detection by the IR temperature sensor device (7) the thickness of the lower side of the housing (41), particularly of the wall of the lower side of the housing (41), is reduced. During use, the analyte concentration is determined based on the analyte sensor signals received by the processor (8) from the transcutaneous analyte sensor (3) and based on the temperature signals received by the processor (8) from the IR temperature sensor device (7).

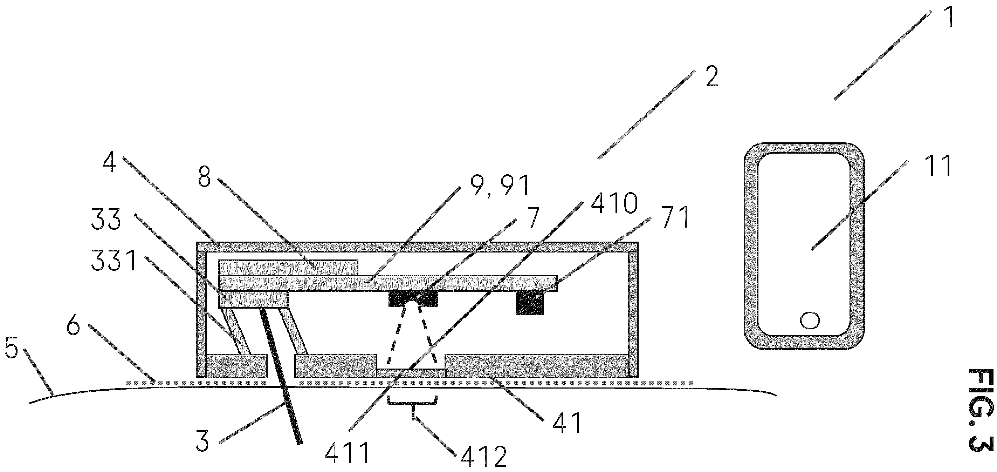

**Figure 3** shows a schematic frontal view of an analyte sensor system (1) according to another embodiment of the invention. This embodiment differs from the one described for Figure 2 only in the following aspects: firstly, the thickness of the lower side of the housing (41, 411), particularly of the the wall of the lower side of the housing (41, 411), is reduced only in the area of the internal surface of the lower side of housing opposite of the surface facing the skin (410), specifically in the temperature detection area of the lower side of housing (412). Secondly, the circuit board is additionally equipped with a contact temperature sensor (71). This way any impact of the temperature of the circuit board (9) can be taken into account that may adversely affect the temperature detection by the IR temperature sensor device (7), if, e.g. the IR temperature sensor device (7) is heated up by the circuit board (9) beyond its approved temperature operating range. Moreover, the temperature sensor signals by the contact temperature sensor (71) also allow for calibrating the temperature measurement by the IR temperature sensor device (7) to compensate for changing temperatures of the IR temperature sensor device (7) and its IR temperature sensor.

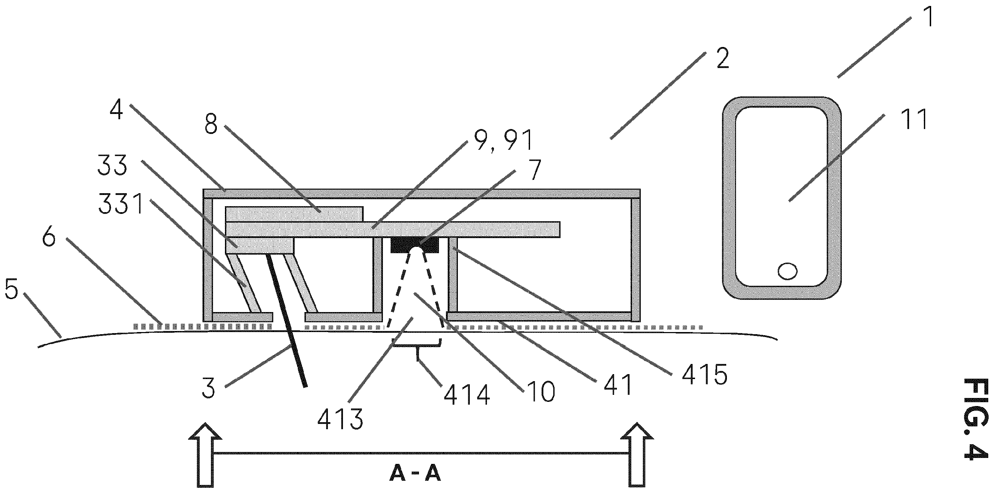

**Figure 4** shows a schematic frontal view of an analyte sensor system (1) according to another embodiment of the invention. This embodiment differs from the one described for Figure 2 only in the following aspect: the lower side of the housing comprises a hole (413) which defines temperature detection area of the skin (414) configured such that the temperature of the skin surface below the hole can be detecteded directly by the IR temperature sensor device (7), in particular without obstruction by any physical object located between the IR temperature sensor and the skin (5) exposed by the hole (413). Preferably, the plaster (6) is also removed in this area so as to expose the skin (5) surface to the IR temperature sensor device (7). To prevent that dirt, humidity or body liquids reach the circuit board or other parts of the interior of the housing a detection cell (10) is provided which comprises the IR temperature sensor device (7), the lower side of the housing comprising the hole, and separating means (415), preferably separating walls, which seal off the detection cell (10) from the remaining interior space of the housing (4). The bottom- view of the body wearable analyte sensor device (2) indicated by A-A is depicted in Figure 5.

**Figure 5** shows a schematic bottom-up along with a frontal view of an analyte sensor system (1) according to the embodiment of the invention shown in Figure 4. In addition to the frontal view the figure also shows the bottom-up view schematic (upper part of the Figure marked A - A). The measurment cell (10) is separated from the remainder of the interior housing by separating means (415) such as a wall which is depicted in a circular shape but which of course could also take any other shape such as a square shape. Preferably, the separating means (415) are are watertight and thus effectivily prevent an ingress of humidity, dirt and fluid into the remaining space of the interior housing sealed off by the separating means (415).

### Example - Correction function for determination of glucose concentration based on the IR temperature sensor device determined temperature and the glucose sensor signal

Artificial interstitial fluid (AIF) can be obtained from a manufacturer (e.g. Simulated interstitial fluid, BZ254, from biochemazone.com). Alternatively, AIF can be prepared: 2.5 mM CaCl2, 10mM Hepes, 3.5 mM KCl, 0.7 mM MgSO4, 123 mM NaCl, 1.5 mM NaH2PO4, 7.4 mM saccharose is mixed, and the solution is adjusted to pH 7.5. Milli-Q water (18.2 MΩ cm, Millipore, Bedford, MA, USA) (see e.g. "Minimally Invasive Glucose Monitoring Using a Highly Porous Gold Microneedles-Based Biosensor: Characterization and Application in Artificial Interstitial Fluid", Paolo Bollella et al. Catalysts 2019, 9(7), 580; https://doi.org/10.3390/catal9070580). The AIF is then spiked with glucose to yield a defined glucose concentration of e.g. of 100 mg/dl.

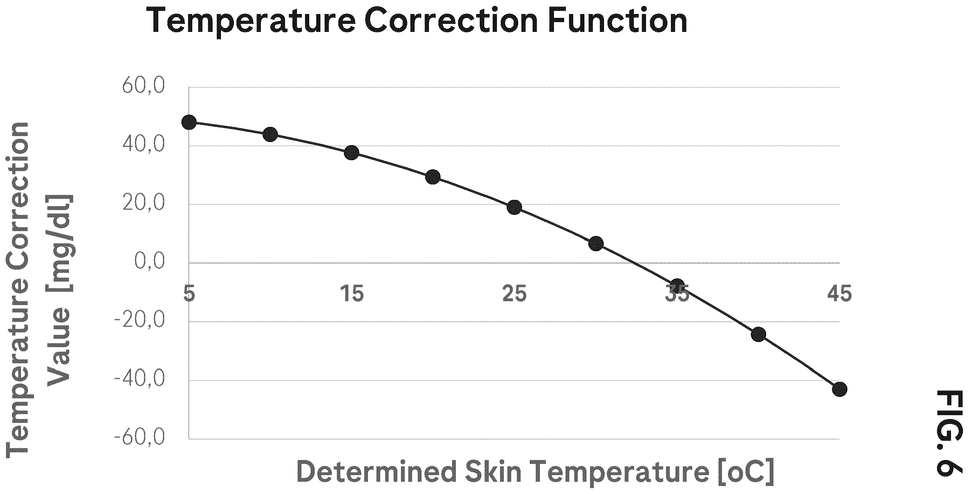

A simulated chitosan/agarose hydrogel skin model (see Bollella et al., supra) is embedded in the glucose spiked AIF and the concentration of the glucose in the hydrogel is determined to be 100 mg/dl (YD1). The skin model is placed into a petri dish. Next a body wearable transcutaneous glucose sensor device of the invention is placed onto a simulated chitosan/agarose hydrogel skin model embedded in the AIF such that the transcutaneous glucose sensor is placed within the hydrogel. Moreover, a thermometer, i.e. a contact temperature sensor, is placed into the hydrogel measure the temperature (X1) of the hydrogel independently from the temperature (X) measured by the IR (non-contact) temperature sensor. The setup is placed in an temperature controlled incubator. The temperature of the incubator is set such that the temperature measured by the IR temperature sensor (X) is 5, 10, 15, 20, 25, 30, 35, and 45 °C. The temperature (X) is determined by the IR temperature temperature sensor of the body wearable transcutaneous glucose sensor device. For each determined temperature x the glucose concentration detected by the transcutaneous glucose sensor (YD2) is recorded and the correction value (YC) is calculated by subtracting the predefined glucose concentration of the hydrogel (YD1) from the glucose concentration YD2 detected by the glucose sensor system. Based on the value pairs X and YC a glucose concentration correction value function can be determined which using the values in table 1 below. The function is $YC = - 0 , 0412 {X}^{2} - 0 , 2163 X + 50 , 13$ (see Figure 6).

This function can then be used by the glucose analyte sensor system of the invention to determining an analyte concentration based on a detected glucose sensor detection signal (YD2) and the determined temperature by the IR temperature tempeature sensor (X). For example, if the glucose sensor detection signal indicates a concentration of YD2=143,8 mg/dl, based on the correction function a correction value of YC= 43,8 mg/dl is calculated and subtracted from YD2 to result in the determined glucose concentration of 100 mg/dl.

**Table 1**

| **Comparison Measurement** | **Determined skin temperature (X)** | **Correction value (YC = YD2 - YD1)** | **Determined glucose concentration (YD2)** | **Set glucose concentration (YD1)** |
|---|---|---|---|---|
| **Nr.** | **[°C]** | **[mg/dl]** | **[mg/dl]** | **[mg/dl]** |
| 1 | 5 | 48,0 | 148,0 | 100 |
| 2 | 10 | 43,8 | 143,8 | 100 |
| 3 | 15 | 37,6 | 137,6 | 100 |
| 4 | 20 | 29,3 | 129,3 | 100 |
| 5 | 25 | 19,0 | 119,0 | 100 |
| 6 | 30 | 6,6 | 106,6 | 100 |
| 7 | 35 | -7,9 | 92,1 | 100 |
| 8 | 40 | -24,4 | 75,6 | 100 |
| 9 | 45 | -43,0 | 57,0 | 100 |

In analogy to the above described glucose concentration correction value function, empirical determination of value pairs of the temperature (X1) of the hydrogel determined by a contact temperature sensor and the correspondingly determined temperature (X) determined by the IR temperature sensor at different temperatures of the hydrogel can be used to calculate a temperature correction value function which can be used to calibrate and correct for temperature measurement deviations by the IR temperature sensor vis-à-vis the thermometor (contact sensor) based temperature measurement of the hydrogel.

It is clear to the skilled person that this methodology can also be applied to analyte sensor systems of the invention detecting analytes other than glucose.

### List of reference numbers

- 1: analyte sensor system
- 2: body wearable analyte sensor device
- 3: transcutaneous analyte sensor
- 33: transcutaneous analyte sensor holder
- 331: separating walls
- 4: housing
- 41: lower side of housing
- 410: internal surface of the lower side of housing opposite of the surface facing the skin
- 411: lower side of housing with reduced thickness
- 412: temperature detection area of the lower side of housing
- 413: hole
- 414: temperature detection area of the skin
- 415: separating means
- 5: skin
- 6: plaster
- 7: IR temperature sensor device comprising a IR temperature sensor
- 71: contact temperature sensor
- 72: glue
- 8: processor
- 81: memory
- 9: electronic unit
- 91: circuit board
- 10: detection cell
- 11: display device

## Claims

1. An analyte sensor system (1) which comprises
• a body wearable analyte sensor device (2), comprising
∘ a transcutaneous analyte sensor (3),
∘ a housing (4) comprising a lower side (41) which can be attached to the skin (5) of a patient, o an infrared (IR) temperature sensor device (7) which is configured to detect the temperature of the skin (5) through a hole (413) comprised by the lower side of the housing (41), respectively,
o wherein the IR temperature sensor device (7) faces without contact the skin, and
• an electronic unit (9) comprising a processor (8) configured to receive analyte sensor signals from the transcutaneous analyte sensor (3) and temperature sensor signals from the IR temperature sensor device (7);
wherein the housing comprises a contact temperature sensor (71), wherein the contact temperature sensor (71) (i) is part of the IR temperature sensor device, or (ii) is not part of the IR temperature sensor device (7) and in contact with a part of the housing carrying the IR temperature sensor device (7) or with the circuit board (91), optionally the contact temperature sensor (71) is selected from the group consisting of a thermoelement, a thermistor, and a resistance temperature dectector;
wherein the processor (8) comprises a memory (81), wherein the processor (8) is configured to receiving the temperature sensor signals from the IR temperature sensor device (7), and determining the temperature of the skin (5) based on the received temperature sensor signals from the IR temperature sensor (7) and from the contact temperature sensor (71); and wherein the processor is configured to calibrate the temperature sensor signals from the IR temperature sensor with the temperature sensor signals from the contact temperature sensor and temperature sensor calibration data to determine the temperature of the skin.

2. An analyte sensor system (1) according to claim 1, wherein the IR temperature sensor device (7) is mounted on a circuit board (91), preferably on a printed circuit board.

3. An analyte sensor system (1) according to claim 1 or 2, wherein the IR temperature sensor device (7) comprises an IR temperature sensor, optionally a passive infrared (PIR) sensor.

4. An analyte sensor system (1) according to any one of claims 1 to 3, wherein the IR temperature sensor device (7) faces without contact the skin (5), wherein the IR temperature sensor device (7) does not contact the lower side of the housing (41) and also not the skin (5), wherein the lower side (41) of the housing comprises the hole (413) defining a temperature detection area of the skin (414, 5) configured such that the temperature of the skin (5) below the hole (413) can be detected by the IR temperature sensor device (7).

5. An analyte sensor system (1) according to claim 4, wherein a detection cell (10) comprises the IR temperature sensor device (7), the lower side of the housing (41) comprising the hole (413), and separating means (415), sealing off the detection cell (10) from the remaining interior space of the housing (4).

6. An analyte sensor system (1) according to any one of claims 1 to 5, wherein the analyte sensor system (1) further comprises a display device (11) configured to communicate with the body wearable analyte sensor device (2).

7. An analyte sensor system (1) according to any one of claims 1 to 6, wherein the transcutaneous analyte sensor (3) is a glucose sensor.

8. An analyte sensor system (1) according to any one of claims 1 to 7, wherein the processor (8) is further configured to
• receiving the analyte sensor signals from the transcutaneous analyte sensor (3),
• determining an analyte concentration based on the analyte sensor signals and the determined temperature, and
• communicating the analyte concentration to the display device (11).

9. An analyte sensor system (1) according to any one of claims 1 to 8, wherein the processor (8) is further configured to
• comparing the determined temperature with a first and optionally also with a second predetermined reference temperature, and
• issueing a notification if (i) the determined temperature is above the first predetermined reference temperature, and (ii) optionally if the determined temperature is below the second predetermined reference temperature, wherein the first predetermined reference temperature is higher than the second predetermined reference temperature.

10. An analyte sensor system (1) according to any one of claims 1 to 9, wherein
(i) the contact temperature sensor (71) is part of the IR temperature sensor device (7); or
(ii) the contact temperature sensor (71) is not part of the IR temperature sensor device (7) and in contact with a part of the housing carrying the IR temperature sensor device (7) or with the circuit board (91); and
wherein the processor (8) is configured to calibrate the temperature sensor signals from the IR temperature sensor with the temperature sensor signals from the contact temperature sensor (71) and temperature sensor calibration data to determine the temperature of the lower side of the housing (41) or the temperature of the skin (5).

11. An analyte sensor system (1) according to claim 10, wherein the calibrated signals compensate for changing temperatures detected by the contact temperature sensor (71).

## Patentansprüche

1. Analytsensorsystem (1), umfassend:
• eine am Körper tragbare Analytsensorvorrichtung (2), umfassend
∘ einen transkutanen Analytsensor (3),
∘ ein Gehäuse (4), umfassend eine Unterseite (41), die an der Haut (5) eines Patienten befestigt werden kann,
∘ eine Infrarot(IR)-Temperatursensorvorrichtung (7), die konfiguriert ist, um die Temperatur der Haut (5) durch eine Öffnung (413) zu erfassen, die jeweils von der Unterseite des Gehäuses (41) umfasst wird,
∘ wobei die IR-Temperatursensorvorrichtung (7) ohne Kontakt der Haut zugewandt ist, und
• eine elektronische Einheit (9), umfassend einen Prozessor (8), der konfiguriert ist, um Analytsensorsignale von dem transkutanen Analytsensor (3) und Temperatursensorsignale von der IR-Temperatursensorvorrichtung (7) zu empfangen;
wobei das Gehäuse einen Kontakttemperatursensor (71) umfasst, wobei der Kontakttemperatursensor (71) (i) Teil der IR-Temperatursensorvorrichtung ist oder (ii) nicht Teil der IR-Temperatursensorvorrichtung (7) ist und in Kontakt mit einem Teil des Gehäuses, der die IR-Temperatursensorvorrichtung (7) trägt, oder mit der Leiterplatte (91) steht, wobei der Kontakttemperatursensor (71) gegebenenfalls ausgewählt ist aus der Gruppe bestehend aus einem Thermoelement, einem Thermistor und einem Widerstandstemperaturfühler;
wobei der Prozessor (8) einen Speicher (81) umfasst, wobei der Prozessor (8) konfiguriert ist, um die Temperatursensorsignale von der IR-Temperatursensorvorrichtung (7) zu empfangen und die Temperatur der Haut (5) basierend auf den empfangenen Temperatursensorsignalen von dem IR-Temperatursensor (7) und von dem Kontakttemperatursensor (71) zu bestimmen; und wobei der Prozessor konfiguriert ist, um die Temperatursensorsignale von dem IR-Temperatursensor mit den Temperatursensorsignalen von dem Kontakttemperatursensor und Temperatursensorkalibrierungsdaten zu kalibrieren, um die Temperatur der Haut zu bestimmen.

2. Analytsensorsystem (1) nach Anspruch 1, wobei die IR-Temperatursensorvorrichtung (7) auf einer Leiterplatte (91), vorzugsweise auf einer gedruckten Leiterplatte, montiert ist.

3. Analytsensorsystem (1) nach Anspruch 1 oder 2, wobei die IR-Temperatursensorvorrichtung (7) einen IR-Temperatursensor, gegebenenfalls einen passiven Infrarot(PIR)-Sensor, umfasst.

4. Analytsensorsystem (1) nach einem der Ansprüche 1 bis 3, wobei die IR-Temperatursensorvorrichtung (7) ohne Kontakt der Haut (5) zugewandt ist, wobei die IR-Temperatursensorvorrichtung (7) nicht die Unterseite des Gehäuses (41) und auch nicht die Haut (5) berührt, wobei die Unterseite (41) des Gehäuses die Öffnung (413) umfasst, die einen Temperaturerfassungsbereich der Haut (414, 5) definiert, der so konfiguriert ist, dass die Temperatur der Haut (5) unter der Öffnung (413) von der IR-Temperatursensorvorrichtung (7) erfasst werden kann.

5. Analytsensorsystem (1) nach Anspruch 4, wobei eine Erfassungszelle (10) die IR-Temperatursensorvorrichtung (7), die Unterseite des Gehäuses (41), die die Öffnung (413) umfasst, und Trennmittel (415) umfasst, die die Erfassungszelle (10) gegenüber dem verbleibenden Innenraum des Gehäuses (4) abdichten.

6. Analytsensorsystem (1) nach einem der Ansprüche 1 bis 5, wobei das Analytsensorsystem (1) ferner eine Anzeigevorrichtung (11) umfasst, die konfiguriert ist, um mit der am Körper tragbaren Analytsensorvorrichtung (2) zu kommunizieren.

7. Analytsensorsystem (1) nach einem der Ansprüche 1 bis 6, wobei der transkutane Analytsensor (3) ein Glukosesensor ist.

8. Analytsensorsystem (1) nach einem der Ansprüche 1 bis 7, wobei der Prozessor (8) ferner zu Folgendem konfiguriert ist:
• Empfangen der Analytsensorsignale von dem transkutanen Analytsensor (3),
• Bestimmen einer Analytkonzentration basierend auf den Analytsensorsignalen und der bestimmten Temperatur, und
• Kommunizieren der Analytkonzentration an die Anzeigevorrichtung (11).

9. Analytsensorsystem (1) nach einem der Ansprüche 1 bis 8, wobei der Prozessor (8) ferner zu Folgendem konfiguriert ist:
• Vergleichen der bestimmten Temperatur mit einer ersten und gegebenenfalls auch mit einer zweiten vorbestimmten Referenztemperatur und
• Ausgeben einer Benachrichtigung, wenn (i) die bestimmte Temperatur über der ersten vorbestimmten Referenztemperatur liegt, und (ii) gegebenenfalls, wenn die bestimmte Temperatur unter der zweiten vorbestimmten Referenztemperatur liegt, wobei die erste vorbestimmte Referenztemperatur höher als die zweite vorbestimmte Referenztemperatur ist.

10. Analytsensorsystem (1) nach einem der Ansprüche 1 bis 9, wobei
(i) der Kontakttemperatursensor (71) Teil der IR-Temperatursensorvorrichtung (7) ist; oder
(ii) der Kontakttemperatursensor (71) nicht Teil der IR-Temperatursensorvorrichtung (7) ist und in Kontakt mit einem Teil des Gehäuses, der die IR-Temperatursensorvorrichtung (7) trägt, oder mit der Leiterplatte (91) steht; und
wobei der Prozessor (8) konfiguriert ist, um die Temperatursensorsignale von dem IR-Temperatursensor mit den Temperatursensorsignalen von dem Kontakttemperatursensor (71) und Temperatursensorkalibrierungsdaten zu kalibrieren, um die Temperatur der Unterseite des Gehäuses (41) oder die Temperatur der Haut (5) zu bestimmen.

11. Analytsensorsystem (1) nach Anspruch 10, wobei die kalibrierten Signale sich ändernde Temperaturen kompensieren, die von dem Kontakttemperatursensor (71) erfasst werden.

## Revendications

1. Système de capteur d'analyte (1) qui comprend
• un capteur d'analyte vestimentaire (2), comprenant
∘ un capteur d'analyte transcutané (3),
∘ un boîtier (4) comprenant un côté inférieur (41) qui peut être attaché à la peau (5) d'un patient,
∘ un capteur de température infrarouge (IR) (7) qui est configuré pour détecter la température de la peau (5) à travers un orifice (413) compris par le côté inférieur du boîtier (41), respectivement,
∘ dans lequel le capteur de température IR (7) est orienté sans contact vers la peau, et
• une unité électronique (9) comprenant un processeur (8) configuré pour recevoir des signaux de capteur d'analyte provenant du capteur d'analyte transcutané (3) et des signaux de capteur de température provenant du capteur de température IR (7) ;
dans lequel le boîtier comprend un capteur de température de contact (71), dans lequel le capteur de température de contact (71) (i) fait partie du capteur de température IR, ou (ii) ne fait pas partie du capteur de température IR (7) et est en contact avec une partie du boîtier portant le capteur de température IR (7) ou avec la carte de circuit (91), éventuellement le capteur de température de contact (71) est choisi dans le groupe constitué par un thermoélément, une thermistance et un détecteur de température à résistance ;
dans lequel le processeur (8) comprend une mémoire (81), dans lequel le processeur (8) est configuré pour recevoir les signaux de capteur de température provenant du capteur de température IR (7), et déterminer la température de la peau (5) en se basant sur les signaux de capteur de température reçus provenant du capteur de température IR (7) et du capteur de température de contact (71) ; et dans lequel le processeur est configuré pour étalonner les signaux de capteur de température provenant du capteur de température IR avec les signaux de capteur de température provenant du capteur de température de contact et les données d'étalonnage de capteur de température pour déterminer la température de la peau.

2. Système de capteur d'analyte (1) selon la revendication 1, dans lequel le capteur de température IR (7) est monté sur une carte de circuit (91), de préférence sur une carte de circuit imprimé.

3. Système de capteur d'analyte (1) selon la revendication 1 ou 2, dans lequel le capteur de température IR (7) comprend un capteur de température IR, éventuellement un capteur infrarouge passif (PIR).

4. Système de capteur d'analyte (1) selon l'une quelconque des revendications 1 à 3, dans lequel le capteur de température IR (7) est orienté sans contact vers la peau (5), dans lequel le capteur de température IR (7) n'entre pas en contact avec le côté inférieur du boîtier (41) et également pas avec la peau (5), dans lequel le côté inférieur (41) du boîtier comprend l'orifice (413) définissant une zone de détection de température de la peau (414, 5) configurée de telle sorte que la température de la peau (5) sous l'orifice (413) peut être détectée par le capteur de température IR (7).

5. Système de capteur d'analyte (1) selon la revendication 4, dans lequel une cellule de détection (10) comprend le capteur de température IR (7), le côté inférieur du boîtier (41) comprenant l'orifice (413), et un moyen de séparation (415), isolant de manière étanche la cellule de détection (10) de l'espace intérieur restant du boîtier (4).

6. Système de capteur d'analyte (1) selon l'une quelconque des revendications 1 à 5, dans lequel le système de capteur d'analyte (1) comprend en outre un dispositif d'affichage (11) configuré pour communiquer avec le capteur d'analyte vestimentaire (2).

7. Système de capteur d'analyte (1) selon l'une quelconque des revendications 1 à 6, dans lequel le capteur d'analyte transcutané (3) est un capteur de glucose.

8. Système de capteur d'analyte (1) selon l'une quelconque des revendications 1 à 7, dans lequel le processeur (8) est en outre configuré pour
• recevoir les signaux de capteur d'analyte provenant du capteur d'analyte transcutané (3),
• déterminer une concentration en analyte en se basant sur les signaux de capteur d'analyte et la température déterminée, et
• communiquer la concentration en analyte au dispositif d'affichage (11).

9. Système de capteur d'analyte (1) selon l'une quelconque des revendications 1 à 8, dans lequel le processeur (8) est en outre configuré pour
• comparer la température déterminée à une première et éventuellement également à une seconde température de référence prédéterminée, et
• émettre une notification si (i) la température déterminée est supérieure à la première température de référence prédéterminée, et (ii) éventuellement si la température déterminée est inférieure à la seconde température de référence prédéterminée, dans lequel la première température de référence prédéterminée est supérieure à la seconde température de référence prédéterminée.

10. Système de capteur d'analyte (1) selon l'une quelconque des revendications 1 à 9, dans lequel
(i) le capteur de température de contact (71) fait partie du capteur de température IR (7) ; ou
(ii) le capteur de température de contact (71) ne fait pas partie du capteur de température IR (7) et est en contact avec une partie du boîtier portant le capteur de température IR (7) ou avec la carte de circuit (91) ; et
dans lequel le processeur (8) est configuré pour étalonner les signaux de capteur de température provenant du capteur de température IR avec les signaux de capteur de température provenant du capteur de température de contact (71) et les données d'étalonnage de capteur de température pour déterminer la température du côté inférieur du boîtier (41) ou la température de la peau (5).

11. Système de capteur d'analyte (1) selon la revendication 10, dans lequel les signaux étalonnés compensent les changements de température détectés par le capteur de température de contact (71).
